# EUROPÄISCHE PATENTSCHRIFT

(11) **EP 1 135 092 B1**
(45) Veröffentlichungstag und Bekanntmachung des Hinweises auf die Patenterteilung: **03.03.2004**
(21) Anmeldenummer: 99965435.3
(22) Anmeldetag: 03.12.1999
(51) Int. Cl.: A61J 3/10

(54) **TEILBARE FESTE DOSIERUNGSFORMEN UND VERFAHREN ZU IHRER HERSTELLUNG**
CLEAVABLE SOLID DOSAGE FORMS AND METHOD FOR THE PRODUCTION THEREOF
FORMES POSOLOGIQUES SOLIDES SECABLES ET LEUR PROCEDE DE PRODUCTION

(30) Priorität: 04.12.1998 DE 19856147
(43) Veröffentlichungstag der Anmeldung: 26.09.2001
(73) Patentinhaber: Abbott GmbH & Co. KG, 65205 Wiesbaden (DE)
(72) Erfinder: ZEIDLER, Jürgen, D-67112 Mutterstadt (DE); ROSENBERG, Jörg, D-67158 Ellerstadt (DE); MAIER, Werner, D-67105 Schifferstadt (DE); NEUMANN, Jörg, D-67117 Limburgerhof (DE)
(74) Vertreter: Kinzebach, Werner, Dr.
(86) Internationale Anmeldenummer: PCT/EP1999/009463
(87) Internationale Veröffentlichungsnummer: WO 2000/033786

(56) Entgegenhaltungen:
- DE-A- 4 229 085
- DE-A- 4 446 470
- US-A- 4 735 805
- PATENT ABSTRACTS OF JAPAN vol. 1997, no. 02, 28. Februar 1997 (1997-02-28) & JP 08 277217 A (SUMITOMO PHARMACEUT CO LTD), 22. Oktober 1996 (1996-10-22)

## Beschreibung

Die vorliegende Erfindung betrifft teilbare feste Dosierungsformen, insbesondere feste pharmazeutische Dosierungsformen, und ein Verfahren zu ihrer Herstellung.

Häufig ist es erwünscht, dass feste pharmazeutische Dosierungsformen, z. B. Tabletten, teilbar sind, um die Dosierung eines Wirkstoffs variieren zu können, ohne dass für bestimmte Dosierungen jeweils eigene Tabletten hergestellt werden müssen. Die Teilung einer Tablette in genau vorbestimmte Teile gestattet die Verabreichung einer Teilmenge oder eines beliebigen Vielfachen der Teilmenge des in der Tablette enthaltenen wirkstoffs.

Zur Erleichterung der Teilung weisen Tabletten üblicherweise Bruchkerben auf. Das Auseinanderbrechen der Tablette erfolgt, indem auf die Tablette Druck ausgeübt wird, wobei die Tablette zwischen zwei Fingern oder mit beiden Händen gehalten wird. Teilbare Tabletten sind z. B. in der CH 683 066 oder der US 3,927,194 beschrieben.

Die DE-OS 30 30 622 beschreibt eine teilbare Tablette mit kontrollierter und verzögerter Wirkstoffabgabe. Das Verhältnis von Länge zu Breite zu Höhe soll dabei etwa 2,5 bis 5 zu etwa 0,9 bis 2 zu 1 betragen. Es ist eine oder mehrere senkrecht zur Länge und zur Höhe verlaufende relativ tiefe Bruchrille vorhanden. Die Grund- und Deckfläche sind unabhängig voneinander plan oder um die Längsachse oder Parallelen zu dieser konvex gebogen.

Die DE-OS 44 46 470 beschreibt ein Verfahren zur Herstellung teilbarer Tabletten durch Formen einer wirkstoffhaltigen Schmelze in einem Kalander mit zwei gegenläufig rotierenden Formwalzen, die Vertiefungen zur Aufnahme und Formung der Schmelze zu Tabletten aufweisen, wobei die Vertiefungen durch mindestens einen Steg unterteilt sind, der sich im Wesentlichen bis zur Mantelfläche der Formwalze erstreckt und die Formung der Bruchrille bewirkt. US 4,735,805 beschreibt eine Dosierungsform gemäß dem Oberbegriff von Anspruch 1.

Ein Problem bei den bekannten teilbaren festen Dosierungsformen besteht darin, dass ein relativ hoher Kraftaufwand zur Teilung der Dosierungsformen erforderlich ist. Dieses Problem ist bei durch Schmelzextrusion hergestellten Dosierungsformen besonders ausgeprägt, da diese gewöhnlich aus einem sehr harten und spröden Material bestehen. Man hat versucht, dieses Problem zu umgehen, indem man die festen Dosierungsformen mit sehr tiefen Einkerbungen mit großen Kerbwinkeln versehen hat. Bei diesen Lösungsansätzen besteht allerdings die Gefahr, dass die festen Dosierungsformen bei Nachbehandlungsschritten, z. B. beim Entgraten oder Filmcoating, durch die Materialbelastung zerbrechen, was zu einem hohen Ausschussanteil führt.

Ein weiteres großes Problem stellt bei bekannten teilbaren festen Dosierungsformen die unzureichende Masseneinheitlichkeit der manuell geteilten Hälften der Dosierungsformen dar. Entsprechende Untersuchungen ergaben Standardabweichungen der Tablettenhälften zwischen 3 und 13 % (siehe H.G. Kristensen et al., Pharmeuropa, Band 7, Nr. 2, Juni 1995, S. 298 ff.). Derartig hohe Standardabweichungen führen zu ungenauen Dosierungen, was insbesondere bei hochwirksamen Wirkstoffen unerwünscht ist. Die unzureichende Masseneinheitlichkeit der manuell geteilten Tablettenhälften beruht zum einen darauf, dass die Bruchfläche nicht exakt an der vorhergesehenen Stelle verläuft, und zum anderen darauf, dass das Tablettenmaterial an äußeren Kanten der Tablettenform zum Ausbrechen neigt, was zu Wirkstoffverlusten führt.

Der vorliegenden Erfindung liegt daher die Aufgabe zugrunde, feste Dosierungsformen zur Verfügung zu stellen, die eine leichte Teilbarkeit und gleichzeitig eine ausreichende Belastbarkeit für Weiterverarbeitungsschritte aufweisen. Weiterhin sollten die festen Dosierungsformen so gestaltet sein, dass die bei der manuellen Teilung hervorgehenden Bruchteile der festen Dosierungsformen eine möglichst gute Masseneinheitlichkeit aufweisen.

Überraschenderweise wurde gefunden, dass diese Aufgabe durch eine bestimmte Geometrie der festen Dosierungsformen gelöst werden kann. Die vorliegende Erfindung betrifft daher eine feste, längliche Dosierungsform (10) mit einer Längsachse (11) und einer durch Projektion der Enden (12, 13) der Dosierungsform auf die Längsachse definierten Länge (L), wobei eine senkrecht zu der Längsachse (11) orientierte Querschnittsfläche (14, 15, 16) einen entlang der Längsachse (11) variablen Flächeninhalt aufweist, der von einer zwischen den Enden (12, 13) befindlichen Querschnittsfläche (15) mit minimalem Flächeninhalt zu beiden Enden (12, 13) hin im Wesentlichen kontinuierlich bis zu jeweils einer Querschnittsfläche (14 bzw. 16) mit maximalem Flächeninhalt ansteigt, die dadurch gekennzeichnet ist, dass der Abstand der in der Nähe des einen Endes (12) befindlichen maximalen Querschnittsfläche (14) von der in der Nähe des anderen Endes (13) befindlichen maximalen Querschnittsfläche (16) in Projektion auf die Längsachse (11) mehr als die halbe Länge (L) der Dosierungsform (10) beträgt.
- Figur 1: zeigt eine erfindungsgemäße Dosierungsform im Querschnitt.
- Figur 2: zeigt eine Vertiefung in der Formwalze eines Formkalanders, mit dem erfindungsgemäße Dosierungsformen erhalten werden können.
- Figur 3: zeigt eine erfindungsgemäße Dosierungsform, die unter Anwendung einer Formwalze mit Vertiefungen der in Figur 2 gezeigten Art erhalten werden kann.

Als Längsachse wird hier diejenige Hauptträgheitsachse der festen Dosierungsform bezeichnet, auf der die Projektion der Dosierungsform die größte Ausdehnung hat. Diese Ausdehnung wird im Folgenden als Länge (L) der Dosierungsform bezeichnet. Die Ausdehnungen der Projektionen der Dosierungsform auf die beiden anderen Hauptträgheitsachsen entsprechen der Höhe bzw. Breite der Dosierungsform. "Länglich" bedeutet, dass die Länge der Dosierungsform größer als ihre Breite oder ihre Höhe ist. Vorzugsweise beträgt die Länge der Dosierungsform mehr als das 2,5-fache, insbesondere das 3- bis 4,5-fache, der Breite oder Höhe. Unter der Querschnittsfläche wird für die Zwecke der vorliegenden Erfindung die Fläche verstanden, die auf einer senkrecht zur Längsachse der festen Dosierungsform orientierten Ebene von der Schnittlinie der äußeren Oberfläche der Dosierungsform mit der Ebene eingeschlossen wird. Die beiden Querschnittsflächen mit maximalem Flächeninhalt liegen sich bezüglich der Querschnittsflächen mit minimalem Flächeninhalt in einem Abstand von mehr als der halben Länge der Dosierungsform gegenüber. Die erfindungsgemäßen Dosierungsformen können z. B. auf Höhe der minimalen Querschnittsfläche eine allseitige "Einschnürung" aufweisen. Alternativ kann nur an einer Tablettenseite eine Kerbe vorliegen, während die gegenüberliegende Seite plan verläuft. Ferner können erfindungsgemäße Dosierungsformen an gegenüberliegneden Seiten mit Kerben versehen sein.

Üblicherweise nimmt bei den erfindungsgemäßen Dosierungsformen der Flächeninhalt der Querschnittsfläche nach Erreichen der maximalen Querschnittsfläche zu einem oder zu beiden Enden hin wieder ab. Er kann aber auch nach Erreichen der maximalen Querschnittsfläche bis zu den beiden Enden der Dosierungsform auf diesem maximalen Wert konstant bleiben. In bestimmten Ausführungsformen wird der maximale Querschnitt erst an den Enden der Dosierungsform erreicht.

Die Form der erfindungsgemäßen Dosierungsform gestattet eine gute Greifbarkeit der Dosierungsform mit beiden Händen. Die längliche Form mit den außenliegenden Masseschwerpunkten bewirkt eine günstige Drehmomentwirkung, die die Teilung der Dosierungsformen erleichtert. Aufgrund der kleinen Querschnittsfläche, entlang derer die Teilung der Dosierungsform erfolgt, ist eine geringe "Brucharbeit" zu leisten. Die erfindungsgemäßen Dosierungsformen lassen sich in Bruchstücke mit exakt vorherbestimmter Größe teilen. Hierdurch wird eine gute Masseneinheitlichkeit der manuell geteilten Bruchstücke der erfindungsgemäßen Dosierungsformen mit geringer Standardabweichung erreicht. Die erfindungsgemäßen Dosierungsformen können auch sehr gut weiterverarbeitet werden, ohne zu zerbrechen. Aufgrund der durch die Form der erfindungsgemäßen Dosierungsformen vorgegebenen Massenverteilung sind die Dosierungsformen gegenüber axialer Belastung und gegenüber Seitenaufprall weitgehend stabil.

Bevorzugte erfindungsgemäße feste Dosierungsformen sind dadurch gekennzeichnet, dass der Flächeninhalt der minimalen Querschnittsfläche (15) höchstens zwei Drittel, insbesondere höchstens das 0,6-fache, des Flächeninhalts der maximalen Querschnittsfläche beträgt.

Der wichtigste Anwendungsfall der vorliegenden Erfindung sind halbierbare feste Dosierungsformen. Bei diesen Dosierungsformen liegt die minimale Querschnittsfläche in einer Spiegelsymmetrieebene der Dosierungsform.

Bei bevorzugten erfindungsgemäßen Dosierungsformen weist wenigstens eine in Längsrichtung verlaufende Mantellinie der Dosierungsform auf Höhe der minimalen Querschnittsfläche einen Knick auf. Hierdurch wird die Sollbruchstelle der Dosierungsform genauer definiert. Als Maß für den Knick lässt sich ein Kerbwinkel definieren, der dem Winkel entspricht, der von den beiden beidseits der minimalen Querschnittsfläche an die Dosierungsformoberfläche angelegten Tangentialebenen eingeschlossen wird. Bevorzugte erfindungsgemäße Dosierungsformen weisen einen Kerbwinkel von mehr als 90°, insbesondere von mehr als 100°, auf. Der Kerbwinkel ist im Allgemeinen kleiner als 170°, vorzugsweise kleiner als 162°. Eine gegebenenfalls vorhandene, nachstehend erörterte, Bruchrille bleibt hierbei außer Betracht.

Es wurde festgestellt, dass beim Auseinanderbrechen von Dosierungsformen das Tablettenmaterial an scharfen Kanten zum Ausbrechen neigt. Bevorzugte erfindungsgemäße Dosierungsformen weisen daher im Wesentlichen abgerundete Kanten auf. Insbesondere ist bevorzugt, dass die erfindungsgemäßen Dosierungsformen an ihrem Äquator im Wesentlichen keinen Steg aufweisen. Auf diese Weise können Wirkstoffverluste bei der manuellen Teilung der Dosierungsformen minimiert werden.

Zur weiteren Erleichterung der Teilbarkeit kann in der Oberfläche der erfindungsgemäßen Dosierungsform entlang des Umfangs oder entlang von Abschnitten des Umfangs der minimalen Querschnittsfläche eine Bruchrille ausgespart sein. Die Tiefe der Bruchrille ist vorzugsweise klein gegenüber der Tiefe der Einschnürung bzw. der Kerbe, die durch den Unterschied der Querschnittsflächen bedingt ist.

Erfindungsgemäße feste Dosierungsformen können nach einem beliebigen Verfahren hergestellt werden. Es ist jedoch besonders bevorzugt, die Dosierungsformen durch Schmelzkalandrieren herzustellen. Die Schmelzkalandrierung gestattet die Herstellung von Dosierungsformen, die keinen ausgeprägten Tablettensteg aufweisen. Als Steg wird bei Tabletten, die durch übliche Presstablettierung hergestellt sind, die von der Matrizenwand gebildete Fläche bezeichnet.

Bei der Schmelzkalandrierung werden mindestens ein polymeres Bindemittel, mindestens ein Wirkstoff und gegebenenfalls übliche Additive unter Bildung eines plastischen Gemisches vermischt, und dieses Gemisch wird in einem Kalander mit zwei gegenläufig rotierenden Formwalzen geformt. Wenigstens eine der Formwalzen weist dabei Vertiefungen zur Aufnahme und Formung des plastischen Gemisches zu Dosierungsformen auf, wobei die Vertiefungen so ausgestaltet sind, dass feste Dosierungsformen gemäß der obigen Definition erhalten werden.

Die erfindungsgemäßen Dosierungsformen umfassen im Allgemeinen:
a) 0,1 bis 90 Gew.-%, insbesondere 0,1 bis 60 Gew.-% (bezogen auf das Gesamtgewicht der Dosierungsform) eines Wirkstoffes,
b) 10 bis 99,9 Gew.-%, insbesondere 40 bis 99,9 Gew.-% eines Bindemittels, vorzugsweise polymeren Bindemittels und
c) gegebenenfalls Additive.

Als polymere Bindemittel geeignet sind Polymere, Copolymere, Cellulosederivate, Stärke und Stärkederivate, beispielsweise:
Polyvinylpyrrolidon (PVP), Copolymerisate von N-Vinylpyrrolidon (NVP) und Vinylacetat oder Vinylpropionat, Copolymerisate von Vinylacetat und Crotonsäure, teilverseiftes Polyvinylacetat, Polyvinylalkohol, Polyhydroxyalkylacrylate, Polyhydroxyalkylmethacrylate, Polyacrylate und Polymethacrylate (Eudragit-Typen), Copolymerisate von Methylmethacrylat und Acrylsäure, Polyacrylamide, Polyethylenglykole, Polyvinylformamid (gegebenenfalls partiell oder vollständig hydrolysiert), Celluloseester, Celluloseether, insbesondere Methylcellulose und Ethylcellulose, Hydroxyalkylcellulosen, insbesondere Hydroxypropylcellulose, Hydroxyalkyl-Alkylcellulosen, insbesondere Hydroxypropyl-Ethylcellulose, Cellulosephthalate, insbesondere Celluloseacetatphthalat und Hydroxypropylmethylcellulosephthalat, und Mannane, insbesondere Galactomannane. Davon sind Polyvinylpyrrolidon, Copolymerisate von N-Vinylpyrrolidon und Vinylestern, Polyhydroxyalkylacrylate, Polyhydroxyalkylmethacrylate, Polyacrylate, Polymethacrylate, Alkylcellulosen und Hydroxyalkylcellulosen besonders bevorzugt.

Das polymere Bindemittel muß in der Gesamtmischung aller Komponenten im Bereich von 50 bis 180°C, vorzugsweise 60 bis 130°C erweichen oder schmelzen. Die Glasübergangstemperatur der Mischung muss daher unter 180°C, vorzugsweise unter 130°C liegen. Erforderlichenfalls wird sie durch übliche, pharmakologisch akzeptable weichmachende Hilfsstoffe herabgesetzt. Die Menge an Weichmacher beträgt höchstens 30 Gew.-%, bezogen auf das Gesamtgewicht von Bindemittel und Weichmacher, damit lagerstabile Arzneiformen gebildet werden, die keinen kalten Fluss zeigen. Vorzugsweise aber enthält das Gemisch keinen Weichmacher.

Beispiele für derartige Weichmacher sind:
langkettige Alkohole, Ethylenglykol, Propylenglykol, Glycerin, Trimethylolpropan, Triethylenglykol, Butandiole, Pentanole, wie Pentaerythrit, Hexanole, Polyethylenglykole, Polypropylenglykole, Polyethylenpropylenglykole, Silicone, aromatische Carbonsäureester (z. B. Dialkylphthalate, Trimellithsäureester, Benzoesäureester, Terephthalsäureester) oder aliphatische Dicarbonsäureester (z. B. Dialkyladipate, Sebacinsäureester, Azelainsäureester, Zitronen- und Weinsäureester), Fettsäureester, wie Glycerinmono-, Glycerindi- oder Glycerintriacetat oder Natriumdiethylsulfosuccinat. Die Konzentration an Weichmacher beträgt im Allgemeinen 0,5 bis 15, vorzugsweise 0,5 bis 5 Gew.-%, bezogen auf das Gesamtgewicht des Gemisches.

Übliche galenische Hilfsstoffe, deren Gesamtmenge bis zu 100 Gew.-%, bezogen auf das Polymerisat, betragen kann, sind z. B. Streckmittel bzw. Füllstoffe, wie Silikate oder Kieselerde, Magnesiumoxid, Aluminiumoxid, Titanoxid, Methylcellulose, Natrium-Carboxymethylcellulose, Talkum, Saccharose, Lactose, Getreide- oder Maisstärke, Kartoffelmehl, Polyvinylalkohol, insbesondere in einer Konzentration von 0,02 bis 50, vorzugsweise 0,20 bis 20 Gew.-%, bezogen auf das Gesamtgewicht des Gemisches.

Formtrennmittel, wie Magnesium-, Zink- und Calciumstearat, Natriumstearylfumarat, Talkum und Silicone, in einer Konzentration von 0,1 bis 5, vorzugsweise 0,1 bis 3 Gew.-%, bezogen auf das Gesamtgewicht des Gemisches. Als Formtrennmittel geeignet sind weiterhin tierische oder pflanzliche Fette, insbesondere in hydrierter Form und solche, die bei Raumtemperatur fest sind. Diese Fette haben vorzugsweise einen Schmelzpunkt von 50°C oder höher. Bevorzugt sind Triglyceride der C₁₂-, C₁₄-, C₁₆- und C₁₈-Fettsäuren. Auch Wachse, wie Carnaubawachs, sind brauchbar. Diese Fette und Wachse können vorteilhaft alleine oder zusammen mit Mono- und/oder Diglyceriden oder Phosphatiden, insbesondere Lecithin, zugemischt werden. Die Mono- und Diglyceride stammen vorzugsweise von den oben erwähnten Fettsäuretypen ab. Die Gesamtmenge an Fetten, Wachsen, Mono-, Diglyceriden und/oder Lecithinen beträgt 0,1 bis 30, vorzugsweise 0,1 bis 5 Gew.-%, bezogen auf das Gesamtgewicht der Masse für die jeweilige Schicht;
Fließmittel, wie Aerosil (hochdisperses SiO₂) oder Talkum;
Farbstoffe, wie Azofarbstoffe, organische oder anorganische Pigmente oder Farbstoffe natürlicher Herkunft, wobei anorganische Pigmente in einer Konzentration von 0,001 bis 10, vorzugsweise 0,5 bis 3 Gew.-%, bezogen auf das Gesamtgewicht des Gemisches bevorzugt sind;
Stabilisatoren, wie Antioxidanzien, Lichtstabilisatoren, Hydroperoxid-Vernichter, Radikalfänger, Stabilisatoren gegen mikrobiellen Befall.

Ferner können Netz-, Konservierungs-, Spreng- und Adsorptionsmittel zugesetzt werden (vgl. z. B. H. Sucker et al., Pharmazeutische Technologie, Thieme-Verlag, Stuttgart 1978).

Unter Hilfsstoffen im Sinne der Erfindung sind auch Substanzen zur Herstellung einer festen Lösung des Wirkstoffs zu verstehen. Diese Hilfsstoffe sind beispielsweise Pentaerythrit und Pentaerythrit-tetraacetat, Polymere wie z. B. Polyethylen- bzw. Polypropylenoxide und deren Blockcopolymere (Poloxamere), Phosphatide wie Lecithin, Homo- und Copolymere des Vinylpyrrolidons, Tenside wie Polyoxyethylen-40-stearat sowie Zitronen- und Bernsteinsäure, Gallensäuren, Sterine und andere wie z. B. bei J. L. Ford, Pharm. Acta Helv. 61, 69-88 (1986) angegeben.

Als Hilfsstoffe gelten auch Zusätze von Basen und Säuren zur Steuerung der Löslichkeit eines Wirkstoffes (siehe beispielsweise K. Thoma et al., Pharm. Ind. 51, 98-101 (1989)).

Einzige Voraussetzung für die Eignung von Hilfsstoffen sind eine ausreichende Temperaturstabilität sowie eine ausreichende Kompatibilität des Hilfsstoffes mit dem Wirkstoff.

Unter Wirkstoffen im Sinne der Erfindung sind alle Stoffe mit einer physiologischen Wirkung zu verstehen, sofern sie sich unter den Verarbeitungsbedingungen nicht vollständig zersetzen. Es handelt sich insbesondere um pharmazeutische Wirkstoffe (für Mensch und Tier), Wirkstoffe für die Pflanzenbehandlung, Insektizide, Futter- und Nahrungsmittelwirkstoffe, Riechstoffe und Parfümöle. Die Wirkstoffmenge pro Dosiseinheit und die Konzentration können je nach Wirksamkeit und Freisetzungsgeschwindigkeit in weiten Grenzen variieren. Die einzige Bedingung ist, dass sie zur Erzielung der gewünschten Wirkung ausreichen. So kann die Wirkstoffkonzentration im Bereich von 0,1 bis 95, vorzugsweise von 20 bis 80, insbesondere 30 bis 70 Gew.-% liegen. Auch Wirkstoff-Kombinationen können eingesetzt werden. Wirkstoffe im Sinne der Erfindung sind auch Vitamine und Mineralstoffe. zu den Vitaminen gehören die Vitamine der A-Gruppe, der B-Gruppe, worunter neben B₁, B₂, B₆ und B₁₂ sowie Nicotinsäure und Nicotinamid auch Verbindungen mit Vitamin B-Eigenschaften verstanden werden, wie z. B. Adenin, Cholin, Pantothensäure, Biotin, Adenylsäure, Folsäure, Orotsäure, Pangamsäure, Carnitin, p-Aminobenzoesäure, myo-Inosit und Liponsäure sowie Vitamin C, Vitamine der D-Gruppe, E-Gruppe, F-Gruppe, H-Gruppe, I- und J-Gruppe, K-Gruppe und P-Gruppe. Zu Wirkstoffen im Sinne der Erfindung gehören auch Peptidtherapeutika. Zu Pflanzenbehandlungsmitteln zählen z. B. Vinclozolin, Epoxiconazol und Quinmerac.

Das erfindungsgemäße Verfahren ist beispielsweise zur Verarbeitung folgender Wirkstoffe geeignet:
Acebutolol, Acetylcystein, Acetylsalicylsäure, Acyclovir, Alfacalcidol, Allantoin, Allopurinol, Alprazolam, Ambroxol, Amikacin, Amilorid, Aminoessigsäure, Amiodaron, Amitriptylin, Amlodipin, Amoxicillin, Ampicillin, Ascorbinsäure, Aspartam, Astemizol, Atenolol, Beclomethason, Benserazid, Benzalkonium-Hydrochlorid, Benzocain, Benzoesäure, Betamethason, Bezafibrat, Biotin, Biperiden, Bisoprolol, Bromazepam, Bromhexin, Bromocriptin, Budesonid, Bufexamac, Buflomedil, Buspiron, Coffein, Campher, Captopril, Carbamazepin, Carbidopa, Carboplatin, Cefachlor, Cefadroxil, Cefalexin, Cefazolin, Cefixim, Cefotaxim, Ceftazidim, Ceftriaxon, Cefuroxim, Chloramphenicol, Chlorhexidin, Chlor-pheniramin, Chlortalidon, Cholin, Cyclosporin, Cilastatin, Cimetidin, Ciprofloxacin, Cisapride, Cisplatin, Clarithromycin, Clävulansäure, Clomipramin, Clonazepam, Clonidin, Clotrimazol, Codein, Cholestyramin, Cromoglycinsäure, Cyanocobalamin, Cyproteron, Desogestrel, Dexamethason, Dexpanthenol, Dextromethorphan, Dextropropoxiphen, Diazepam, Diclofenac, Digoxin, Dihydrocodein, Dihydroergotamin, Dihydroergotoxin, Diltiazem, Diphenhydramin, Dipyridamol, Dipyron, Disopyramid, Domperidon, Dopamin, Doxycyclin, Enalapril, Ephedrin, Epinephrin, Ergocalciferol, Ergotamin, Erythromycin, Estradiol, Ethinylestradiol, Etoposid, Eucalyptus Globulus, Famotidin, Felodipin, Fenofibrat, Fenoterol, Fentanyl, Flavin-Mononucleotid, Fluconazol, Flunarizin, Fluorouracil, Fluoxetin, Flurbiprofen, Folinsäure, Furosemid, Gallopamil, Gemfibrozil, Gentamicin, Gingko Biloba, Glibenclamid, Glipizid, Clozapin, Glycyrrhiza glabra, Griseofulvin, Guaifenesin, Haloperidol, Heparin, Hyaluronsäure, Hydrochlorothiazid, Hydrocodon, Hydrocortison, Hydromorphon, Ipratropium-Hydroxid, Ibuprofen, Imipenem, Imipramin, Indomethacin, Iohexol, Iopamidol, Isosorbid-Dinitrat, Isosorbid-Mononitrat, Isotretinoin, Itraconazol, Ketotifen, Ketoconazol, Ketoprofen, Ketorolac, Labetalol, Lactulose, Lecithin, Levocarnitin, Levodopa, Levoglutamid, Levonorgestrel, Levothyroxin, Lidocain, Lipase, Lisinopril, Loperamid, Lorazepam, Lovastatin, Medroxyprogesteron, Menthol, Methotrexat, Methyldopa, Methylprednisolon, Metoclopramid, Metoprolol, Miconazol, Midazolam, Minocyclin, Minoxidil, Misoprostol, Morphin, Multivitamin-Mischungen bzw. - Kombinationen und Mineralsalze, N-Methylephedrin, Naftidrofuryl, Naproxen, Neomycin, Nicardipin, Nicergolin, Nicotinamid, Nicotin, Nicotinsäure, Nifedipin, Nimodipin, Nitrazepam, Nitrendipin, Nizatidin, Norethisteron, Norfloxacin, Norgestrel, Nortriptylin, Nystatin, Ofloxacin, Omeprazol, Ondansetron, Pancreatin, Panthenol, Pantothensäure, Paracetamol, Penicillin G, Penicillin V, Pentoxifyllin, Phenobarbital, Phenoxymethylpenicillin, Phenylephrin, Phenylpropanolamin, Phenytoin, Piroxicam, Polymyxin B, Povidon-Iod, Pravastatin, Prazepam, Prazosin, Prednisolon, Prednison, Propafenon, Propranolol, Proxyphyllin, Pseudoephedrin, Pyridoxin, Quinidin, Ramipril, Ranitidin, Reserpin, Retinol, Riboflavin, Rifampicin, Rutosid, Saccharin, Salbutamol, Salcatonin, Salicylsäure, Selegilin, Simvastatin, Somatropin, Sotalol, Spironolacton, Sucralfat, Sulbactam, Sulfamethoxazol, Sulfasalazin, Sulpirid, Tamoxifen, Tegafur, Teprenön, Terazosin, Terbutalin, Terfenadin, Tetracyclin, Theophyllin, Thiamin, Ticlopidin, Timolol, Tranexamsäure, Tretinoin, Triamcinolon-Acetonid, Triamteren, Trimethoprim, Troxerutin, Uracil, Valproinsäure, Vancomycin, Verapamil, Vitamin E, Zidovudin.

Bevorzugte Wirkstoffe sind Ibuprofen (als Racemat, Enantiomer oder angereichertes Enantiomer), Metoprolol, Ketoprofen, Flurbiprofen, Acetylsalicylsäure, Verapamil, Paracetamol, Nifedipin oder Captopril.

Zur Herstellung der festen Dosierungsformen wird ein plastisches Gemisch der Komponenten (Schmelze) bereitgestellt, das anschließend einem Formgebungsschnitt unterzogen wird. Das Vermischen der Komponenten und die Bildung der Schmelze können auf unterschiedliche Weise erfolgen. Das Vermischen kann vor, während und/oder nach der Bildung der Schmelze erfolgen. Beispielsweise können die Komponenten zuerst vermischt und dann aufgeschmolzen oder gleichzeitig vermischt und aufgeschmolzen werden. Häufig erfolgt noch eine Homogenisierung des plastischen Gemisches, um eine hochdisperse Verteilung des Wirkstoffes zu erhalten.

Insbesondere bei Verwendung von empfindlichen Wirkstoffen hat es sich aber als bevorzugt erwiesen, zuerst das polymere Bindemittel, gegebenenfalls zusammen mit üblichen pharmazeutischen Additiven, aufzuschmelzen und vorzuvermischen und dann den (die) empfindlichen Wirkstoff(e) in "Intensivmischern" in plastischer Phase bei sehr kleinen Verweilzeiten einzumischen (Homogenisieren). Der (die) Wirkstoff(e) kann (können) dabei in fester Form oder als Lösung oder Dispersion eingesetzt werden.

Im Allgemeinen werden die Komponenten als solche in das Herstellungsverfahren eingesetzt. Sie können jedoch auch in flüssiger Form, d. h. als Lösung, Suspension oder Dispersion zur Anwendung kommen.

Als Lösungsmittel für die flüssige Form der Komponenten kommt in erster Linie Wasser oder ein mit Wasser mischbares, organisches Lösungsmittel oder ein Gemisch davon mit Wasser in Betracht. Brauchbare Lösungsmittel sind aber auch mit Wasser nicht mischbare oder mischbare, organische Lösungsmittel. Geeignete, mit Wasser mischbare Lösungsmittel sind insbesondere C₁-C₄-Alkanole, wie Ethanol, Isopropanol oder n-Propanol, Polyole, wie Ethylenglykol, Glycerin und Polyethylenglykole. Geeignete, mit Wasser nicht mischbare Lösungsmittel sind Alkane, wie Pentan oder Hexan, Ester, wie Ethylacetat oder Butylacetat, chlorierte Kohlenwasserstoffe, wie Methylenchlorid und aromatische Kohlenwasserstoffe, wie Toluol und Xylol. _Ein weiteres brauchbares Lösungsmittel ist flüssiges CO₂.

Welches Lösungsmittel im Einzelfall verwendet wird, hängt von der aufzunehmenden Komponente und deren Eigenschaften ab. Beispielsweise kommen pharmazeutische Wirkstoffe häufig in Form eines Salzes, das im Allgemeinen wasserlöslich ist, zur Anwendung. Wasserlösliche Wirkstoffe können daher als wässrige Lösung eingesetzt werden oder vorzugsweise in die wässrige Lösung oder Dispersion des Bindemittels aufgenommen werden. Entsprechendes gilt für Wirkstoffe, die in einem der genannten Lösungsmittel löslich sind, wenn die flüssige Form der zur Anwendung kommenden Komponenten auf einem organischen Lösungsmittel basiert.

Gegebenenfalls kann an die Stelle des Aufschmelzens ein Lösen, Suspendieren oder Dispergieren in den oben genannten Lösungsmitteln, falls erwünscht und/oder erforderlich unter Zusatz geeigneter Hilfsstoffe, wie z. B. Emulgatoren, treten. Das Lösungsmittel wird dann im Allgemeinen unter Bildung der Schmelze in einer geeigneten Apparatur, z. B. einem Extruder, entfernt. Im Folgenden soll dies von dem Begriff Vermischen umfasst werden.

Das Aufschmelzen und/oder Vermischen erfolgt in einer für diesen Zweck üblichen Vorrichtung. Besonders geeignet sind Extruder oder gegebenenfalls beheizbare Behälter mit Rührwerk, z. B. Kneter, (wie der unten noch erwähnten Art).

Als Mischapparat sind insbesondere solche Vorrichtungen brauchbar, die in der Kunststofftechnologie zum Mischen eingesetzt werden. Geeignete Vorrichtungen sind beispielsweise beschrieben in "Mischen beim Herstellen und Verarbeiten von Kunststoffen", H. Pahl, VDI-Verlag, 1986. Besonders geeignete Mischapparaturen sind Extruder und dynamische und statische Mischer, sowie Rührkessel, einwellige Rührwerke mit Abstreifvorrichtungen, insbesondere sogenannte Pastenrührwerke, mehrwellige Rührwerke, insbesondere PDSM-Mischer, Feststoffmischer sowie vorzugsweise Misch-Knetreaktoren (z. B. ORP, CRP, AP, DTB der Firma List oder Reactotherm der Firma Krauss-Maffei oder Ko-Kneter der Fa. Buss), Doppelmuldenkneter (Trogmischer) und Stempelkneter (Innenmischer) oder Rotor/Stator-Systeme (z. B. Dispax der Firma IKA).

Bei empfindlichen Wirkstoffen erfolgt vorzugsweise zunächst das Aufschmelzen des polymeren Bindemittels in einem Extruder und anschließend das Zumischen des Wirkstoffs in einem Misch-Knetreaktor. Bei weniger empfindlichen Wirkstoffen kann man dagegen zum intensiven Dispergieren des Wirkstoffs ein Rotor/Stator-System einsetzen.

Das Beschicken der Mischvorrichtung erfolgt je nach deren Konzeption kontinuierlich oder diskontinuierlich in üblicher Weise. Pulverförmige Komponenten können im freien Zulauf, z. B. über eine Differentialdosierwaage eingeführt werden. Plastische Massen können direkt aus einem Extruder eingespeist oder über eine Zahnradpumpe, die insbesondere bei hohen Viskositäten und hohen Drüken von Vorteil ist, zugespeist werden. Flüssige Medien können über ein geeignetes Pumpenaggregat zudosiert werden.

Das durch Vermischen und/oder Aufschmelzen des Bindemittels, des Wirkstoffes und gegebenenfalls des Additivs oder der Additive erhaltene Gemisch ist teigig bis zähflüssig (thermoplastisch) oder flüssig und daher extrudierbar. Die Glasübergangstemperatur des Gemisches liegt unter der Zersetzungstemperatur aller in dem Gemisch enthaltenen Komponenten. Das Bindemittel soll vorzugsweise in physiologischer Umgebung löslich oder quellbar sein.

Die Verfahrensschritte Vermischen und Aufschmelzen können in derselben Apparatur oder in zwei oder mehreren getrennt arbeitenden Vorrichtungen ausgeführt werden. Die Zubereitung einer Vormischung kann in einer der oben beschriebenen üblichen Mischvorrichtungen durchgeführt werden. Eine solche Vormischung kann dann direkt, z. B. in einen Extruder, eingespeist und anschließend gegebenenfalls unter Zusatz weiterer Komponenten extrudiert werden.

Das erfindungsgemäße Verfahren erlaubt es, als Extruder Einschneckenmaschinen, kämmende Schneckenmaschinen oder auch Mehrwellenextruder, insbesondere Zweischnecken-Extruder, gleichsinnig oder gegensinnig drehend und gegebenenfalls mit Knetscheiben ausgerüstet, einzusetzen. Wenn bei der Extrusion ein Lösungsmittel verdampft werden muss, sind die Extruder im Allgemeinen mit einem Verdampfungsteil ausgerüstet. Besonders bevorzugt sind Extruder der ZSK-Baureihe von Werner u. Pfleiderer.

Das erhaltene Gemisch ist vorzugsweise lösungsmittelfrei, d. h. es enthält weder Wasser noch ein organisches Lösungsmittel.

Das plastische Gemisch wird einer Formgebung in einem Kalander mit gegenläufig rotierenden Formwalzen unterzogen. Die Formwalzen weisen auf ihren Oberflächen Vertiefungen auf, die zur Aufnahme und Formung des plastischen Gemisches geeignet sind. Das plastische Gemisch wird dabei in den trogähnlichen Raum zwischen den Formwalzen, z. B. mittels eines Füllkeils, eingefüllt. An der Berührungslinie der Formwalzen treten kurzzeitig jeweils zwei einander entsprechende Vertiefungen auf den Formwalzen zu einer Tablettenform zusammen. Bei der Weiterrotation streben die Vertiefungen wieder auseinander und geben die geformte Dosierungsform frei. Die Vertiefungen auf den Formwalzen sind so ausgestaltet, dass sie der Negativform einer "Hälfte" der erfindungsgemäßen Dosierungsformen entsprechen. Einander entsprechende Vertiefungen auf den Formwalzen können gleiche Form oder unterschiedliche Form haben. Die Vertiefungen auf einer Formwalze können eine gleichförmige Tiefe aufweisen, während die Vertiefungen der anderen Formwalze entlang ihrer Längsachse unterschiedliche Tiefen aufweist. Alternativ können die Vertiefungen in beiden Formwalzen entlang der Längsachsen variierende Tiefen aufweisen. Es ist bevorzugt, dass die Längsachse der Vertiefungen parallel zur Rotationsachse der Formwalzen liegt.

Die erhaltenen Dosierungsformen können in einem nachgeschalteten Verfahrensschritt nach üblichen Verfahren gerundet, entgratet und/oder mit einem Coating versehen werden. Geeignete Materialien für Filmüberzüge sind Filmbildner, wie z. B. Polyacrylate, wie die Eudragit-Typen, Celluloseester, wie die Hydroxypropylcellulosephthalate, sowie Celluloseether, wie Ethylcellulose, Hydroxypropylmethylcellulose oder Hydroxypropylcellulose, gegebenenfalls in Abmischung mit Füllmitteln, Farbpigmenten, Benetzungsmitteln und Weichmachern.

Im Einzelnen kann es zur Ausbildung von festen Lösungen kommen. Der Begriff "feste Lösungen" ist dem Fachmann geläufig, beispielsweise aus der eingangs zitierten Literatur. In festen Lösungen von Wirkstoffen in Polymeren liegt der Wirkstoff molekulardispers im Polymer vor.

Die Figuren und das nachstehende Beispiel sollen die Erfindung näher veranschaulichen.

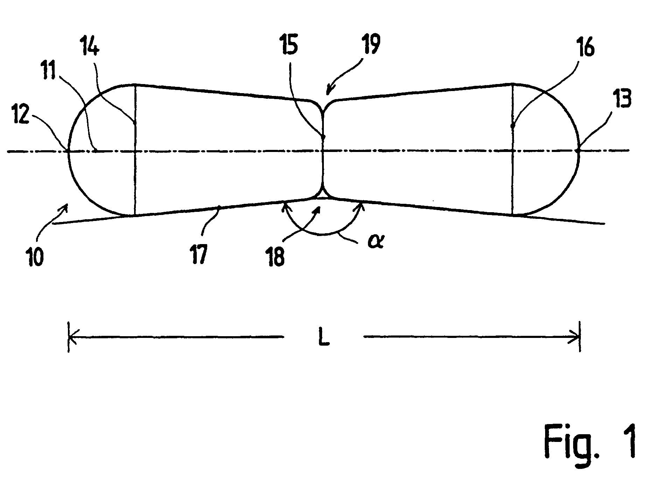

Figur 1 zeigt eine erfindungsgemäße Dosierungsform im Querschnitt. Die Dosierungsform (10) weist eine Längsachse (11) und zwei Enden (12, 13) auf. In der Nähe des einen Endes (12) befindet sich eine erste maximale Querschnittsfläche (14), in der Nähe des anderen Endes (13) eine zweite maximale Querschnittsfläche (16). In der Mitte der Tablette ist eine Querschnittsfläche (15) mit minimalem Querschnitt angeordnet. Die in der Zeichenebene liegende Mantelebene (17) weist auf Höhe der minimalen Querschnittsfläche (15) einen Knick (18) auf. Durch die beidseits der minimalen Querschnittsfläche (15) an die Dosierungsformoberfläche angelegten Tangentialebenen wird ein Kerbwinkel (α) eingeschlossen. Die dargestellte Dosierungsform weist außerdem eine Bruchrille (19) auf.

### Beispiele

### Beispiel 1

Es wurde gemäß folgender Rezeptur eine pharmazeutische Mischung hergestellt:

| | |
|---|---|
| Verapamil-HCl | 48,0 Gew.-%; |
| Hydroxypropylcellulose | 31,5 Gew.-%; |
| Hydroxypropylmethylcellulose | 17,5 Gew.-%; |
| Lecithin | 3,0 Gew.-%. |

Die pharmazeutische Mischung wurde in einem Zweischnecken-Extruder unter folgenden Bedingungen extrudiert:

| | |
|---|---|
| Temperaturbereich | 80-125°C |
| Schneckendrehzahl | 120 U/min |
| Vakuum | 100 mbar |
| Massenfluß | 120 kg/h |

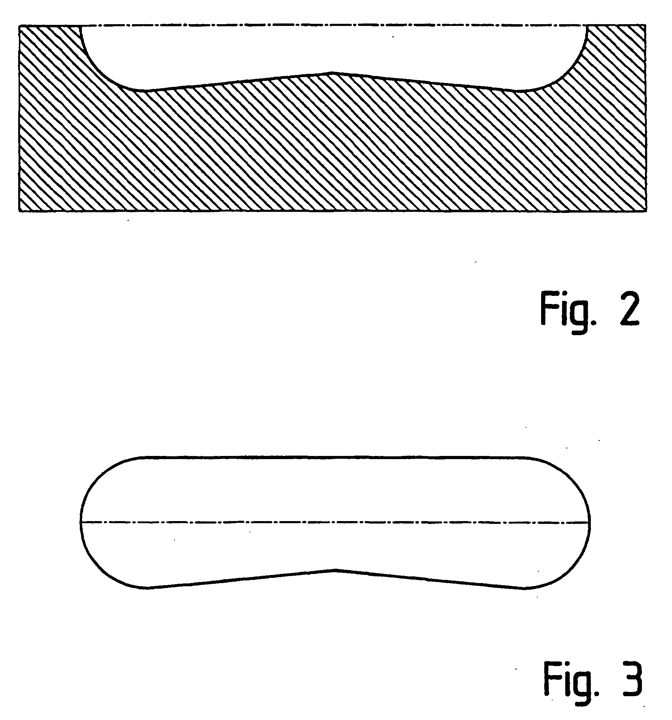

Die Schmelze wurde in einen Formkalander mit zwei Formwalzen geführt. Eine der Formwalzen wies auf ihrer Oberfläche Vertiefungen der in Figur 2 gezeigten Art auf. Die zweite Formwalze wies auf ihrer Oberfläche längliche Vertiefungen mit entsprechender Umfangslinie und einheitlicher Tiefe auf. Man erhielt auf diese Weise Tabletten, wie sie in Figur 3 abgebildet sind. Sie waren leicht und glatt in zwei gleiche Hälften zu zerbrechen. Die ermittelten Standardabweichungen bei den manuell geteilten Dosierungsformen lagen im Bereich von 2 % bei einer Gesamtmasse der Dosierungsformen von 500 mg. Die somit erreichte Standardabweichung liegt in einem pharmazeutisch akzeptablen Bereich.

## Patentansprüche

1. Feste, längliche Dosierungsform (10) mit einer Längsachse (11) und einer durch Projektion der Enden (12, 13) der Dosierungsform auf die Längsachse definierten Länge (L), wobei eine senkrecht zu der Längsachse (11) orientierte Querschnittsfläche (14, 15, 16) einen entlang der Längsachse (11) variablen Flächeninhalt aufweist, der von einer zwischen den Enden (12, 13) befindlichen Querschnittsfläche (15) mit minimalem Flächeninhalt zu beiden Enden (12, 13) hin im Wesentlichen kontinuierlich bis zu jeweils einer Querschnittsfläche (14 bzw. 16) mit maximalem Flächeninhalt ansteigt, wobei der Abstand der in der Nähe des einen Endes (12) befindlichen maximalen Querschnittsfläche (14) von der in der Nähe des anderen Endes (13) befindlichen maximalen Querschnittsfläche (16) in Projektion auf die Längsachse (11) mehr als die halbe Länge (L) der Dosierungsform (10) beträgt und die Dosierungsform an ihrem Äquator keinen Steg aufweist.

2. Dosierungsform nach Anspruch 1, **dadurch gekennzeichnet, daß** der Abstand der in der Nähe des einen Endes (12) befindlichen maximalen Querschnittsfläche (14) von der in der Nähe des anderen Endes (13) befindlichen maximalen Querschnittsfläche (16) in Projektion auf die Längsachse mehr als das 0,6-fache der Länge (L) beträgt.

3. Dosierungsform nach Anspruch 1 oder 2, **dadurch gekennzeichnet, daß** der Flächeninhalt der minimalen Querschnittsfläche (15) höchstens zwei Drittel des Flächeninhalts der maximalen Querschnittsfläche (14, 16) beträgt.

4. Dosierungsform nach Anspruch 3, **dadurch gekennzeichnet, daß** der Flächeninhalt der minimalen Querschnittsfläche (15) höchstens das 0,6-fache des Flächeninhalts der maximalen Querschnittsfläche (14, 16) beträgt.

5. Dosierungsform nach einem der vorhergehenden Ansprüche, **dadurch gekennzeichnet, daß** die minimale Querschnittsfläche (15) in einer Spiegelsymmetrieebene der Dosierungsform liegt.

6. Dosierungsform nach einem der vorhergehenden Ansprüche, **dadurch gekennzeichnet, daß** wenigstens eine in Längsrichtung verlaufende Mantellinie (17) der Dosierungsform (10) auf Höhe der minimalen Querschnittsfläche (15) einen Knick (18) aufweist.

7. Dosierungsform nach Anspruch 6, **dadurch gekennzeichnet, dass** die Dosierungsform einen Kerbwinkel (α) von mehr als 90° aufweist.

8. Dosierungsform nach einem der vorhergehenden Ansprüche, **dadurch gekennzeichnet, dass** sie im Wesentlichen abgerundete Kanten aufweist.

9. Dosierungsform nach einem der vorhergehenden Ansprüche, **dadurch gekennzeichnet, dass** in der Oberfläche der Dosierungsform (10) entlang des Umfangs oder entlang von Abschnitten des Umfangs der minimalen Querschnittsfläche (15) eine Bruchrille (19) ausgespart ist.

10. Verfahren zur Herstellung von Dosierungsformen durch Formen einer wirkstoffhaltigen Schmelze in einem Kalander mit zwei gegenläufig rotierenden Formwalzen, von denen wenigstens eine Vertiefungen zur Aufnahme und Formung der Schmelze zu Dosierungsformen aufweist, **dadurch gekennzeichnet, dass** die Formwalze(n) Vertiefungen aufweist (aufweisen), die so ausgestaltet sind, dass Dosierungsformen nach einem der vorhergehenden Ansprüche erhalten werden.

## Claims

1. A solid, elongate dosage form (10) with a long axis (11) and with a length (L) which is defined by projection of the ends (12, 13) of the dosage form onto the long axis, where a cross-sectional area (14, 15, 16) oriented perpendicular to the long axis (11) has an area which is variable along the long axis (11) and increases from a cross-sectional area (15) which is located between the ends (12, 13) and has a minimal area essentially continuously toward the two ends (12, 13) up to in each case a cross-sectional area (14 and 16 respectively) with a maximal area, where the distance of the maximal cross-sectional area (14) which is located near one end (12) from the maximal cross-sectional area (16) which is located near the other end (13) is, projected on the long axis (11), more than half the length (L) of the dosage form (10) and the dosage form has no band in its equator.

2. A dosage form as claimed in claim 1, wherein the distance of the maximal cross-sectional area (14) located near one end (12) from the maximal cross-sectional area (16) located near the other end (13) is, projected on the long axis, more than 0.6 times the length (L).

3. A dosage form as claimed in claim 1 or 2, wherein the area of the minimal cross-sectional area (15) is not more than two thirds of the area of the maximal cross-sectional area (14, 16).

4. A dosage form as claimed in claim 3, wherein the area of the minimal cross-sectional area (15) is no more than 0.6 times the area of the maximal cross-sectional area (14, 16).

5. A dosage form as claimed in any of the preceding claims, wherein the minimal cross-sectional area (15) lies in a plane of symmetry of the dosage form.

6. A dosage form as claimed in any of the preceding claims, wherein at least one surface line (17) running in the longitudinal direction on the dosage form (10) has a kink (18) at the level of the minimal cross-sectional area (15).

7. A dosage form as claimed in claim 6, wherein the dosage form has a notch angle (α) of more than 90°.

8. A dosage form as claimed in any of the preceding claims, which has essentially rounded edges.

9. A dosage form as claimed in any of the preceding claims, wherein a score (19) is formed in the surface of the dosage form (10) along the periphery or along sections of the periphery of the minimal cross-sectional area (15).

10. A process for producing dosage forms by molding an active ingredient-containing melt in a calender with two counter-rotating molding rolls, of which at least one has depressions to receive and shape the melt to dosage forms, wherein the molding roll(s) has (have) depressions designed so that dosage forms as claimed in any of the preceding claims are obtained.

## Revendications

1. Moule de dosage (10) rigide allongé avec un axe longitudinal (11) et une longueur (L) définie par projection des extrémités (12, 13) du moule de dosage sur l'axe longitudinal, une surface de section (14, 15, 16) orientée perpendiculairement à l'axe longitudinal (11) présentant une superficie variable le long de l'axe longitudinal (11) qui augmente pour l'essentiel continuellement vers les deux extrémités (12, 13) depuis une surface de section (15) située entre les extrémités (12, 13) avec une superficie minimale jusqu'à à chaque fois une surface de section (14 ou 16) avec une superficie maximale, la distance entre la surface de section (14) maximale située à proximité de l'une des extrémités (12) et la surface de section (16) maximale située à proximité de l'autre extrémité (13) étant, en projection sur l'axe longitudinal, supérieure à la moitié de la longueur L du moule de dosage (10), et le moule de dosage ne présentant pas de nervure au niveau de son équateur.

2. Moule de dosage selon la revendication 1, **caractérisé en ce que** la distance entre la surface de section (14) maximale située à proximité de l'une des extrémités (12) et la surface de section (16) maximale située à proximité de l'autre extrémité (13) est, en projection sur l'axe longitudinal, supérieure à 0,6 fois la longueur (L).

3. Moule de dosage selon la revendication 1 ou 2, **caractérisé en ce que** la superficie de la surface de section (15) minimale est d'au maximum deux tiers de la superficie de la surface de section (14, 16) maximale.

4. Moule de dosage selon la revendication 3, **caractérisé en ce que** la superficie de la surface de section (15) minimale est d'au maximum 0,6 fois la superficie de la surface de section (14, 16) maximale.

5. Moule de dosage selon l'une quelconque des revendications précédentes, **caractérisé en ce que** la surface de section (15) minimale se situe dans un plan de symétrie réflexive du moule de dosage.

6. Moule de dosage selon l'une des revendications précédentes, caractérisé en qu'au moins une génératrice (17) du moule de dosage (10) s'étendant en direction longitudinale présente un coude (18) au niveau de la surface de section (15) minimale.

7. Moule de dosage selon la revendication 6, **caractérisé en ce que** le moule de dosage présente un angle d'entaille (α) supérieur à 90°.

8. Moule de dosage selon l'une quelconque des revendications précédentes, **caractérisé en ce qu'**il présente des arêtes pour l'essentiel arrondies.

9. Moule de dosage selon l'une quelconque des revendications précédentes, **caractérisé en ce qu'**une rainure de rupture (19) est ménagée dans la surface du moule de dosage (10), le long de la périphérie ou le long de portions de la périphérie de la surface de section (15) minimale.

10. Procédé de fabrication de moules de dosage par moulage d'une masse fondue contenant des agents actifs dans une calandre avec deux cylindres de moulage à rotation opposée, dont au moins l'un présente des creux pour recevoir et mouler la masse fondue en forme de moules de dosage, **caractérisé en ce que** le(s) cylindre(s) de moulage présente(nt) des creux conçus pour permettre d'obtenir des moules de dosage selon l'une quelconque des revendications précédentes.
